# EUROPEAN PATENT SPECIFICATION

(11) **EP 0 758 252 B1**
(45) Date of publication and mention of the grant of the patent: **03.09.2008**
(21) Application number: 95917088.7
(22) Date of filing: 21.04.1995
(51) Int. Cl.: A61K 51/00, A61P 35/00, A61P 19/08

(54) **TIN-117m-CONTAINING RADIOTHERAPEUTIC AGENTS**
RADIOTHERAPEUTISCHE MITTEL ENTHALTEND ZIN-117M
AGENTS RADIOTHERAPEUTIQUES CONTENANT DE L'ETAIN 117m

(30) Priority: 02.05.1994 US 237003
(43) Date of publication of application: 19.02.1997
(73) Proprietor: Brookhaven Science Associates, Upton, NY 11973-5000 (US)
(72) Inventor: SRIVASTAVA, Suresh, C., Setauket, NY 11733 (US); MEINKEN, George, E., Middle Island, NY 11953 (US); MAUSNER, Leonard, F., Stony Brook, NY 11790 (US); ATKINS, Harold, L., Setauket, NY 11733 (US)
(74) Representative: Dörries, Hans Ulrich
(86) International application number: PCT/US1995/004875
(87) International publication number: WO 1995/029706

(56) References cited:
- US-A- 4 533 541
- CHEMICAL ABSTRACTS, vol. 117, no. 14, 5 October 1992 Columbus, Ohio, US; abstract no. 139439, MAUSNER, L. F. ET AL 'Improved specific activity of reactor produced tin-117m with the Szilard-Chalmers process' & APPL. RADIAT. ISOT. (1992), 43(9), 1117-22 CODEN: ARISEF;ISSN: 0883-2889, 1992
- DATABASE BIOSIS BIOSCIENCES INFORMATION SERVICE, PHILADELPHIA, PA, US AN=93:374256, & SEMIN. ONCOL. , vol. 20, no. 3, 1993 pages 22-26, HOLMES R. A. 'RADIOPHARMACEITICALS IN CLINICAL TRIALS.'
- JOURNAL OF NUCLEAR MEDICINE, vol. 35, no. 5, 1994 NEW YORK US, page 37P ATKINS H. L. ET ALL. 'PALLIATION OF BONE PAIN WITH SN-117M (4+)DTPA'
- RADIOLOGY, vol. 186, no. 1, January 1993 pages 279-283, HAROLD L. ATKINS 'BIODISTRIBUTION OF SN-117M(4+)DTPA FOR PALLIATIVE THERAPY OF PAINFUL OSSEOUS METASTASES.'

## Description

### 1. Field of the Invention

This invention relates to pharmaceutical compositions comprising tin-117m (Sn-117m) stannic complexes with certain chelators, and uses of such pharmaceutical compositions in the preparation of a pharmaceutical which is administered in a defined dose range to alleviate bone pain and to treat osseous metastatic disease. The compositions according to the invention comprise a dosage of from about 10 mCi to about 50 mCi Sn-117m per 70 kilograms body weight.

### 2. Description of the Prior Art

Primary cancer of the bone and osseous metastases derived from tumors arising elsewhere (such as lung, prostate and breast carcinoma, among others) can result in substantial pain. Approximately 60-80% of patients with prostate or breast cancer, and up to about 50% of lung cancer patients, develop bone metastases during the course of their illness. These three types of cancer comprise almost 80% of the 400,000 patients who develop bone metastases each year. More than half of these patients experience severe, chronic bone pain as a result of their metastatic disease.

The alleviation of such bone pain is highly desirable, for both human reasons and as an adjunct to proper effective clinical management of neoplastic disease. Conventional methods of alleviating such severe bone pain include treatment with narcotic analgesics and other pain-killers. Such methods are unsatisfactory because they do not clinically address the source of the pain, and because of the effects of such powerful drugs on the patient's personality, mental state and level of consciousness.

A number of radiopharmaceutical agents have been used for the palliation of bone pain from metastatic lesions. Among these are phosphorus-32 (Joshi et. al., 1965, J. Amer. Med. Assoc. 193: 621-623), strontium-89 chloride (Robinson et al., 1987, Nucl. Med. Biol. 14: 219-222), samarium-153 EDTMP (Logan et al., 1987, J. Nucl. Med. 28: 505-509), rhenium-186 HEDP (Maxon et al., 1990, Radiology 176: 155-159), and iodine-131 hydroxybenzylidene diphosphonate (HBDP) (Eisenhut et al., 1986, J. Nucl. Med. 27: 1255-1261). One of these, strontium-89 chloride, was approved by the FDA for commercial distribution in June, 1993.

Generally, the use of internally-administered radiotherapeutic compositions can result in significant toxicity due to destruction of non-cancerous tissues, particularly bone marrow. Treatment with known radiopharmaceutical agents has been limited because of undesirable side effects resulting from uncontrolled irradiation. In the treatment of osseous metastases, a particular limitation has been that the amount of radiation absorbed by red marrow must be sufficiently low so as to escape myelosuppression or compromise of the stem cells of the hematopoietic system, *i*.*e*., the red and white blood cells. Radiation damage to the bone marrow is typically the result of a lower-than-desired ratio of absorbed radiation in the bone tumor compared with the bone marrow ratio, resulting in bone marrow toxicity at therapeutic dosages. This is a major limitation to the use of ³²P-containing radiopharmaceuticals for treating bone metastases.

It would, therefore, be advantageous to be able to utilize a radiopharmaceutical composition and a treatment which produce minimal toxicity, but provide highly effective, reproducible results.

A particularly useful radioisotope for localization in bone is tin-117m. This radioisotope of tin is easily and economically prepared, either in a cyclotron or in a nuclear reactor, and is readily available. It has a half-life of 14 days and emits a conversion electron having energies (and hence, bone marrow cell-killing capacity) significantly below that of other radioisotopes used in conventional bone radiotherapy. Moreover, the average range (in water) of such conversion electron is from 2- to 10-fold less than the range of β-particle emissions of ³²P, ¹⁸⁶Re, ¹⁵³Sm, ¹³¹I or ⁸⁹Sr. Finally, Sn-117m also emits gamma radiation that is detectable by conventional scintigraphic imaging cameras.

The preparation of tin-117m-labeled stannic (Sn⁴⁺) chelates which localize to bone, and their use as diagnostic radiopharmaceuticals, is described in U.S. Patent No. 4,533,541 (issued on August 6, 1985 to Srivastava et al.) This reference describes the preparation of Sn-117m stannic complexes of methylene diphosphonate (MDP), pyrophosphate (PYP), ethylidenehydroxydisodium phosphonate (EHDP), and diethylenetriaminepentaacetic acid (DTPA) and the usefulness of such compositions as radiodiagnostic agents for scintigraphic imaging of skeletal bone.

Srivastava *et al*. describe the preferential localization of Sn-117m stannic complexes in bone to the substantial exclusion of uptake by blood, muscle, kidney, or liver.

Oster et al., 1985, Int. J. Nucl. Med. Biol. 12: 175-184 describe autoradiographic studies showing that the Sn-117m stannic complexes localize to cortical bone but not bone marrow.
* Atkins et al., 1993, Radiology, 186(1): 279-283 disclose the favorable biodistribution of Sn-117m DTPA complexes in patients suffering from osseous metastases.

There remains a need in the medical arts for an effective treatment for pain resulting from osseous metastases. Although its use for scintigraphic imaging was known in the prior art, there is no indication of any composition or treatment with Sn-117 stannic complexes to alleviate pain associated with cancer in human skeletal bone, or to treat cancer in human skeletal bone.

### SUMMARY OF THE INVENTION

The present invention provides pharmaceutical compositions for the effective clinical management and alleviation of bone pain associated with osseous metastases and other bone tumors. The invention provides stannic chelates of tin-117m useful for alleviating such severe bone pain. The pharmaceutical compositions of the invention are also useful and effective for radiotherapeutic treatment of osseous metastases and other bone tumors.

In a first aspect, the invention provides a pharmaceutical composition comprising a Sn-117m (Sn⁴⁺) chelate complex and a pharmaceutically-acceptable carrier for preparing a medicament useful in palliating pain in skeletal bone associated with cancer in a human. In a preferred embodiment, Sn-117m is complexed with methylenediphosphonate (MDP), pyrophosphate (PYP), ethylidinehydroxysdisodium phosphonate (EDHP), or most preferably diethylene triaminepentaacetic acid (DTPA), or mixtures thereof. The pharmaceutical compositions of the invention are comprised of Sn-117m having a specific activity of Sn-117m of about 0.1 mCi/mg to about 80 Ci/mg. In preferred embodiments, the specific activity of Sn-117m in the pharmaceutical compositions of the invention is from about 2 to about 20 mCi/mg Sn-117m, more preferably from about 2 to about 17 mCi/mg Sn-117m, and most preferably from about 2 to about 12 mCi/mg Sn-117m.

In the use of the pharmaceutical compositions of the invention to alleviate or palliate pain associated with osseous metastases, the pharmaceutical compositions of the invention are administered at a dosage of from about 10 to about 50 mCi Sn-117m, more preferably from about 10 to about 30 mCi Sn-117m, most preferably from about 12 to about 25 mCi Sn-117m per 70 kilograms body weight. This is equivalent to a dosage range of from about 7 mCi to about 130 mCi, more preferably from about 7 mCi to about 80 mCi, and most preferably from about 9 to about 65 mCi.

The most preferred embodiment of the pharmaceutical compositions of the invention comprise Sn⁴⁺-117m chelated with DTPA. In particular formulations of such preferred embodiments, the pharmaceutical composition comprises about a ten-fold to about a 45-fold molar excess of DTPA relative to total tin, about a 15-fold to about a 30-fold molar excess of DTPA relative to total tin, or most preferably about a twenty-fold molar excess of DTPA relative to total tin.

In yet other embodiments of this aspect of the invention, the pharmaceutical compositions comprising Sn⁴⁺-117m chelates and a pharmaceutically acceptable carrier also comprise a toxicity control agent which comprises a divalent calcium ion. The inclusion of such toxicity control agents in the pharmaceutical compositions of the invention act to reduce *in vivo* toxicity associated with administration of the Sn-117m (Sn⁴⁺) chelate complex. The most preferred toxicity control agent is calcium chloride dihydrate (CaCl₂·2H₂O). In further preferred embodiments, the toxicity control agent comprises about 60% to about 90% of the molar amount of the stannic chelating group. More preferably, the toxicity control agent comprises about 70% to about 90% of the molar amount of the stannic chelating group. Most preferably, the toxicity control agent comprises about 80% of the molar amount of stannic chelating group in the composition.

In a second aspect, the invention provides pharmaceutical compositions for use in a method for palliating pain associated with osseous metastases and primary osseous neoplastic disease, comprising the step of administering to a human a pharmaceutical composition of the invention. In a preferred embodiment, the pharmaceutical compositions administered to the human to palliate bone pain comprise a Sn⁴⁺-117m DTPA chelate having a specific activity of about 2 to about 12 mCi/mg, comprised of about a twenty-fold molar excess of DTPA to total tin and further comprising a toxicity control agent that is a divalent calcium ion in an amount that is about 80% of the molar amount of DTPA in the composition, administered at a dosage of from about 12 to about 25 mCi per 70 kilograms body weight, corresponding to from about 9 to about 65 mCi administered dose.

In a third aspect, the invention provides a pharmaceutical composition comprising a Sn-117m (Sn⁴⁺) chelate complex and a pharmaceutically-acceptable carrier for preparing a medicament useful in radiotherapeutic treatment of primary and metastatic neoplastic lesions in skeletal bone in a human. In a preferred embodiment, Sn-117m is complexed with methylenediphosphonate (MDP), pyrophosphate (PYP), ethylene dihydroxydisodium phosphonate (EDHP), or most preferably diethylene triaminepentaacetic acid (DTPA), or mixtures thereof. The pharmaceutical compositions of the invention are comprised of Sn-117m having a specific activity of Sn-117m of about 0.1 mCi/mg to about 80 Ci/mg. In preferred embodiments, the specific activity of Sn-117m in the pharmaceutical compositions of the invention is from about 2 to about 20 mCi/mg Sn-117m, more preferably from about 2 to about 17 mCi/mg Sn-117m, and most preferably from about 2 to about 12 mCi/mg Sn-117m.

In the use of the pharmaceutical compositions of the invention to treat primary or metastatic neoplastic disease in skeletal bone in a human, the pharmaceutical compositions of the invention are administered at a dosage of from about 10. to about 50 mCi Sn-117m, more preferably from about 10 to about 30 mCi Sn-117m, most preferably from about 12 to about 20 mCi Sn-117m per 70 kilograms body weight. This is equivalent to a dosage range of from about 7 mCi to about 130 mCi, more preferably from about 7 mCi to about 80 mCi, and most preferably from about 9 to about 65 mCi.

The most preferred embodiment of the pharmaceutical compositions of the invention for treating primary or metastatic neoplastic disease in skeletal bone in a human comprise Sn⁴⁺-117m chelated with DTPA. In particular formulations of such preferred embodiments, the pharmaceutical composition comprises about a ten-fold to about a 45-fold molar excess of DTPA relative to total tin, about a 15-fold to about a 30-fold molar excess of DTPA relative to total tin, or most preferably about a twenty-fold molar excess of DTPA relative to total tin.

In yet other embodiments of this aspect of the invention, the pharmaceutical compositions comprising Sn⁴⁺-117m chelates and a pharmaceutically acceptable carrier also comprise a toxicity control agent. The inclusion of such toxicity control agents in the pharmaceutical compositions of the invention act to reduce *in vivo* toxicity associated with administration of the Sn-117m (Sn⁴⁺) chelate complex. The toxicity control agents comprise a divalent calcium ion. The most preferred toxicity control agent is calcium chloride dihydrate (CaCl₂·H₂O). In further preferred embodiments, the toxicity control agent comprises about 60% to about 90% of the molar amount of the stannic chelating group. More preferably, the toxicity control agent comprises about 70% to about 90% of the molar amount of the stannic chelating group. Most preferably, the toxicity control agent comprises about 80% of the molar amount of stannic chelating group in the composition.

In a fourth aspect, the invention provides pharmaceutical compositions for use in a method for radiotherapeutic treatment of primary or metastatic osseous neoplastic disease in a human, comprising the step of administering to a human a pharmaceutical composition of the invention. In a preferred embodiment, the pharmaceutical compositions administered to the human in radiotherapeutic treatment protocols comprise a Sn⁴⁺-117m DTPA chelate having a specific activity of about 2 to about 12 mCi/mg, comprised of about a twenty-fold molar excess of DTPA to total tin and further comprising a toxicity control agent that is a divalent calcium ion in an amount that is about 80 % of the molar amount of DTPA in the composition, administered at a dosage of from about 20 to about 50 mCi per 70 kilograms body weight.

In yet another aspect of the invention is provided pharmaceutical compositions for use in methods for palliating skeletal bone pain associated with primary or metastatic osseous neoplastic disease, wherein the amount of the analgesic agent comprising the pharmaceutical compositions of the invention localized at neoplastic sites is monitored by gamma scintigraphy.

The invention also provides kits for preparing the radiopharmaceutical compositions of the invention. Kits for forming complexes between Sn⁴⁺-117m and tin chelators comprise a sealed vial containing a predetermined quantity of the tin chelator.

Specific preferred embodiments of the present invention will become evident from the following more detailed description of certain preferred embodiments and the claims.

### BRIEF DESCRIPTION OF THE DRAWINGS

Figure 1 shows the efficacy of the radioimaging capabilities of the present invention at palliative and treatment dosage levels.
Figure 2 also shows the imaging capability of the present invention at levels which can also be used as a palliative and as treatment.
Figure 3 is a graph which depicts the effect of the method and composition of the present invention on white blood cells and platelets.
Figure 4 is a graph of colony-forming unit activity versus administered dose of Sn⁴⁺-117m DTPA and ³²P.

### DETAILED DESCRIPTION OF THE PREFERRED EMBODIMENT

This invention provides pharmaceutical compositions comprising stannic chelates of Sn-117m for providing relief, alleviation or palliation, of bone pain by administration of the pharmaceutical compositions of the invention. The invention also provides pharmaceutical compositions for use in methods for the treatment of bone cancer, including osseous metastases, by administration of a pharmaceutical composition of the invention, such treatment resulting in remission, regression or cure of the disease. The invention also provides pharmaceutical compositions for use in a method for the combined treatment of pain resulting from cancer in bone, while at the same time permitting monitoring of patients status through imaging of the distribution of the composition of the invention in bone tissue. The compositions according to the invention comprise a dosage of from about 10 mCi to about 50 mCi Sm-117m per 70 kilograms body weight.

As used herein, the term "tin chelator" and "tin chelating agent" is intended to encompass any one of a variety of chemical moieties, or mixtures thereof, capable of effectively chelating stannous and/or stannic tin metal ions and forming a stable chelate with tin under physiological conditions.

As used herein, the term "toxicity control agent" is a divalent calcium ion capable of inhibiting or retarding the effects, primarily the hypocalcemic effects, of administration of unchelated tin chelating agents as part of the pharmaceutical compositions of the invention. It is also capable of preventing formation of complexes between such chelating agents and any mono- or divalent metallic cation present *in vivo* and necessary or beneficial to the physical well-being or metabolic integrity of a human to whom the pharmaceutical compositions of the invention are administered.

The physical characteristics of Sn-117m and other radionuclides are shown in following Table I. In contradistinction to the other nuclides show, Sn-117m is not a beta (β) particle emitter. Instead, Sn-117m decays by isomeric transition with the emission of abundant (114%) conversion electrons of specific energy (127-129 and 152 KeV). These conversion electrons of Sn-117m have a range which is sufficient

**TABLE I**

| PHYSICAL CHARACTERISTICS OF RADIONUCLIDES | | | | | |
|---|---|---|---|---|---|
| Nuclide | Maximum | Average | Average¹ | Half-Life | Gamma |
| | Eβ | Eβ | Range | | Photons |
| | (MeV) | (MeV) | (mm) | (days) | (MeV (%)) |
| Sr-89 | 1.46 | 0.583 | 2.4 | 50.5 | None |
| Re-186 | 1.08 | 0.349 | 1.05 | 3.71 | 0.137 (9.2) |
| Sm-153 | 0.81 | 0.234₂ | 0.55 | 1.93 | 0.103 (28) |
| P-32 | 1.71 | 0.695 | 3.00 | 14.3 | None |
| Sn-117m | 0.127³ | | 0.21⁴ | 13.6 | 0.159 (86) |
| | 0.152³ | | 0.29⁴ | | |

| | | | | | |
|---|---|---|---|---|---|
| ¹ In water, Health Physics and Radiological Handbook, Nuclear Lectern Associates (1984) ² Weighted Average of 3 β energies (E_{βMAX}: 0.64, 0.71, 0.82 Mev) ³ Monoenergetic conversion electron ⁴ Discrete travel of emitted conversion electron (not an average) | | | | | |

for irradiating tumor bearing bone, while imparting a much smaller dose to most of the marrow. The other nuclides shown in the Table have an average range which is significantly longer than the limit range of the conversion electrons of Sn-117m. For example, when the marrow damage from comparable radiation doses of P-32 and Sn-117m was evaluated using the mouse spleen colony forming unit technique, the measurement indicated that P-32 is approximately 28 times more toxic to marrow than Sn-117m at therapy levels. (Mausner et al., 1989, J. Nucl. Med. 30: 1754) Thus, the toxicity of effective amounts of Sn-117m chelate complexes is substantially lower than that which occurs in the use of the other nuclides.

It is also illustrated in the Table that a principal photon of 158.6 keV is also present in 86.4% of the disintegrations of Sn-117m. The emitted photon (gamma, γ) enables effective monitoring of the biodistribution of the Sn-117m by external gamma imaging (planar of SPECT). The other nuclides either lack photon emission or emit photons in abundances which are lower than desirable for conventional gamma imaging methods.

Thus, it is also a feature of the invention that palliation of bone pain and concomitant body imaging can be performed. Furthermore, tumors and especially metastases can advantageously be treated and monitored with certain radioisotope preparations to result in regression, remission or cure of the disease.

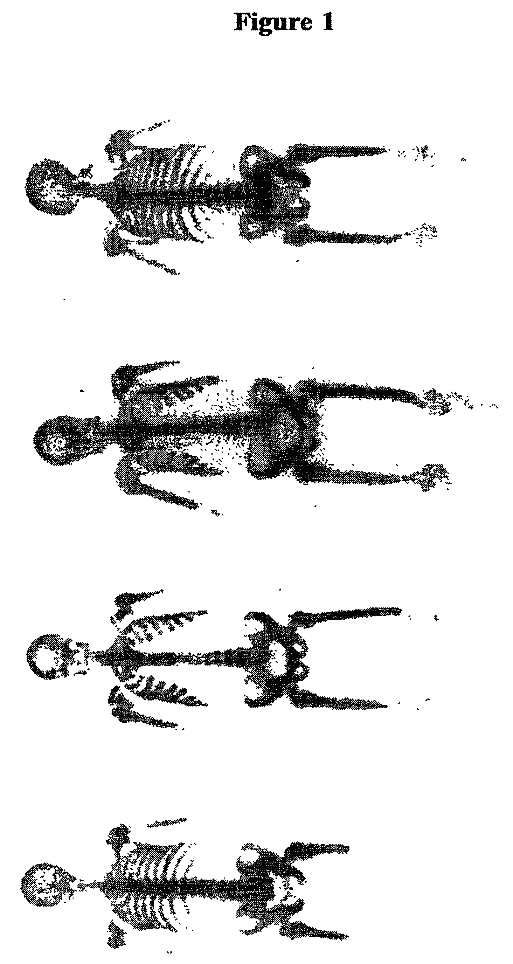

For example, Figure 1 shows Gamma camera images in a patient with prostate cancer that had spread to bone. The left panel shows anterior (right) and posterior (left) of an image of technetium-99m-MDP, the conventional bone scanning agent. The right panel shows anterior (left) and posterior (right) images in the same patient obtained 4 days following injection of 10 mCi of Sn-117m (Sn⁴⁺) DTPA. Note the similarity of distribution of the two nuclides. Also note that the dose of the Sn-117m compound is within the range of dosages found to palliate pain according to the uses of the invention.

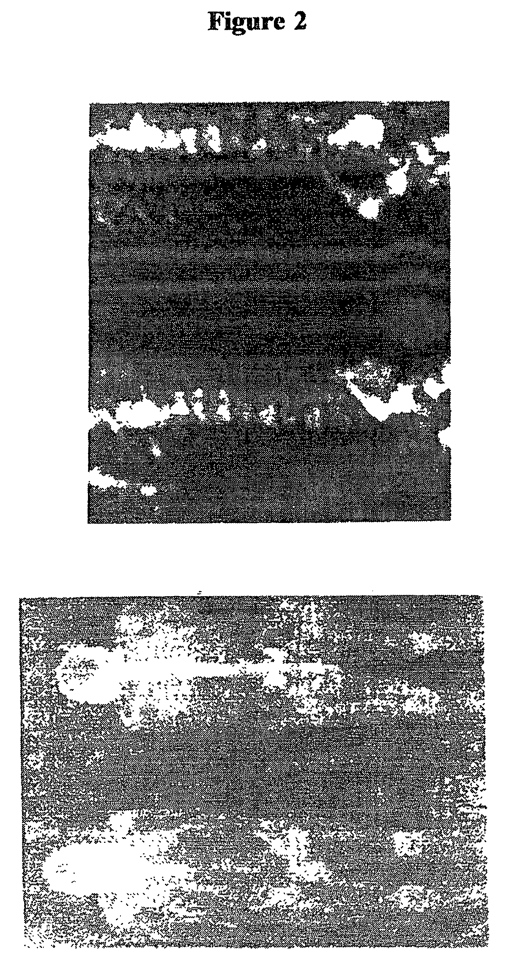

Figure 2 shows images obtained 4 days following injection of 5 mCi of Sn-117m DTPA in a cancer patient with bone metastases. The left panel shows anterior (left) and posterior (right) whole body images of the Sn-117m distribution in bone. The right panel depicts a three-dimensional reprojection image from SPECT (Single Photon Emission Computed Tomography) of a portion of the skeleton in the same patient. The patchy bright spots denote spread of cancer to bone.

Sn-117m is prepared as described in Example 1 below by an inelastic neutron scattering reaction. Targets are irradiated with neutrons from a nuclear reactor for from about 1 to about 10 weeks, preferably from about 2 to about 5 weeks, and most preferably for about 3 weeks, yielding Sn-117m with a specific activity of 2-8 mCi/mg. With a half-life of just under two weeks (as shown in Table I above), production of sufficient amounts of Sn-117m for radiotherapeutic purposes is economically feasible.

The Sn-117m chelates comprised in the pharmaceutical compositions of the invention are prepared from metallic Sn-117m. The metal is first dissolved in a minimal amount of concentrated hydrochloric acid, and then diluted with water to yield a stannous chloride solution in 1-4N HCl. Excess chelator, in 8- to 40-fold molar excess, is added and the pH adjusted to between pH 4 and pH 7 using sodium hydroxide. After complex formation is achieved, the tin metal is oxidized to the 4+ oxidation state (to the stannic form) with hydrogen peroxide.

The Sn⁴⁺-117m chelate thus prepared is used to prepare the pharmaceutical compositions of the invention. The pharmaceutical compositions are formulated in aqueous solution optionally containing pharmaceutically acceptable salts such as sodium chloride, buffers such as sodium or potassium salts of Tris, citrate and acetate, and pharmaceutically acceptable excipients and preservatives and the like.

The pharmaceutical compositions of the invention are administered by a route appropriate to the pathology to be treated, preferably by intravenous injection or infusion in a volume of from about 0.1 to 100 mL solution, diluted with a pharmacologically acceptable diluent such as physiological saline, if desired.

The pharmaceutical compositions provided by this invention are comprised of stannic tin-117m having a specific activity from between about 0.1 mCi/mg to about 80 Ci/mg, administered at a dosage of about 10 mCi to about 50 mCi per 70 kilograms body weight, corresponding to a dosage of from about 7 mCi to about 130 mCi. The composition is comprised of about a 10- to about a 45- a molar excess of the chelating agent, for example and most preferably DTPA, to fully chelate the tin. Optionally and advantageously, a toxicity control agent, most preferably CaCl₂·2H₂O, comprises the pharmaceutical composition in the amount of about 80% of the molar amount of DTPA, limiting the chelation of calcium from blood upon administration to a subject, and thereby avoiding hypocalcemia. A preferred embodiment of the final composition of the pharmaceutical compositions of the invention (having a specific activity of Sn⁴⁺-117m of 2 mCi/mg) is as follows: 10-50 mCi Sn⁴⁺-117m; 5-25 mg Sn⁴⁺-117m; 335-1700 mg DTPA and 100-500 mg CaCl₂-2H₂0, based on a patient having a body weight of 70 kg. For higher specific activity preparation, these ingredients are adjusted in a proportionate manner.

Sn-117m is advantageous for palliating skeletal bone pain and treating primary and metastatic osseous neoplastic disease because it has been found by the present inventors to localize specifically to skeletal bone with low bone marrow toxicity. Results of biodistribution studies are shown in Table II below.

In studies on human cancer patients, Sn⁴⁺117m DTPA was found to specifically localize to skeletal bone. Skeletal uptake averaged 64 ± 13% of the administered dose, having a residence time in bone of 280 ± 65 h. In contrast, the residence time in the remainder of the body was 18 ± 10 h.

Of even greater importance was the finding that bone lesions, comprising metastatic prostate, breast or lung cancer, preferentially accumulated the radiolabel: the activity ratio between bone lesion and normal bone was typically about 5:1.

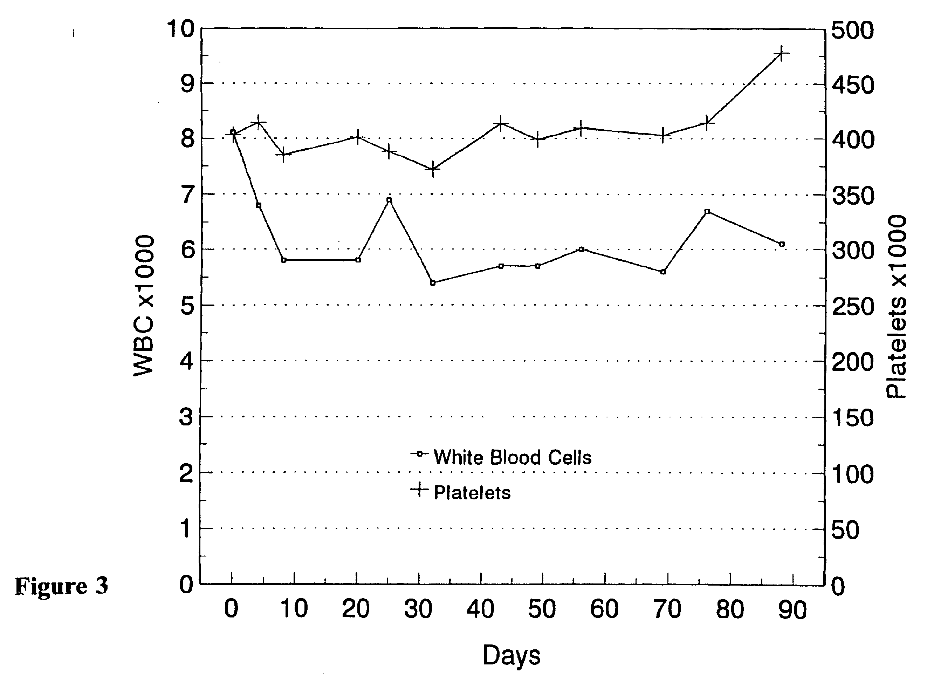

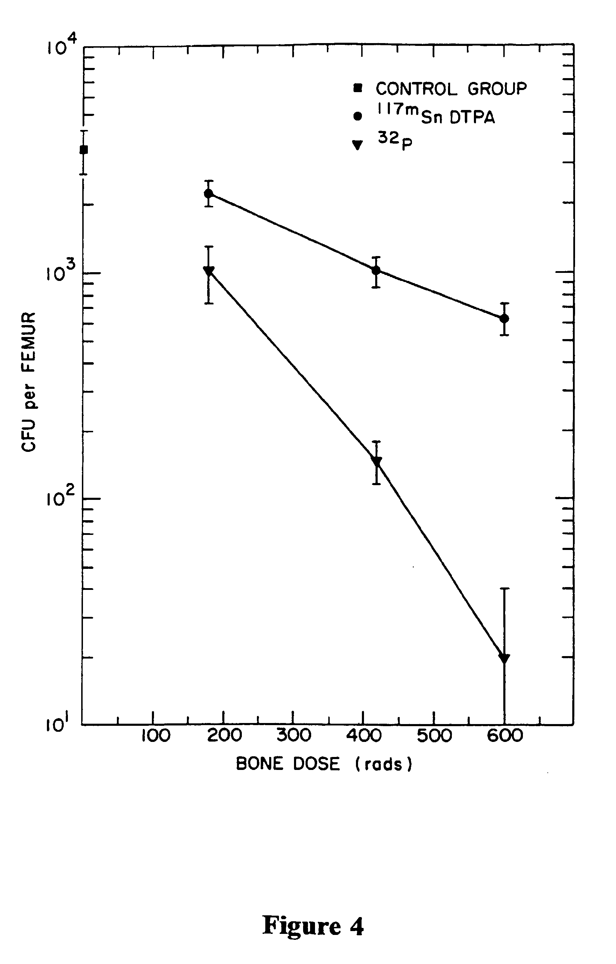

Particularly important is the relative lack of bone marrow toxicity found in animals treated with the pharmaceutical compositions of the invention. For example, Figures 3 and 4 shows the effects of treatment with Sn-117m DTPA compared with conventional treatments using ³²P bone marrow as assayed with a standard colony forming unit assay. In quantitative studies, the bone surface dose was estimated to be 205 ± 50 rad/mCi, while the marrow dose was about 11 rad/mCi. These results demonstrate that Sn⁴⁺-117m DTPA localizes efficiently and specifically to bone, more particularly to bone lesions such as metastatic tumors and indicate the herein disclosed clinical uses of the pharmaceutical compositions of the invention.

As a result of the invention, the excruciating pain associated with cancer in human skeletal bone has been dramatically reduced by treatment with an agent which selectively attacks the source of the pain. Consequently, the treating physician is armed with an effective pain-management tool which is substantially less debilitating

**Table II RADIATION DOSE AND ADMINISTERED ACTIVITY FOR RADIONUCLIDES IN THERAPY OF OSSEOUS METASTASES**

| Nuclide | Surface Bone Dose (rad/mCi) | Red Marrow Dose (rad/mCi) | Ratio Bone: Bone Marrow | Tumor Dose (rad/mCi) | Approximate Therapy Dose (mCi) |
|---|---|---|---|---|---|
| Sr-89 | 58 | 70-104 | 0.8 | 422-882 | 2.8-4.2 |
| Re-186 | 2.9 | 2.8 | 1.0 | 45-890 | 25-35 |
| Sm-153 | 10.5-11.3 | 3.8 | 2.9 | 42.4 | 35 |
| P-32 | 48 | 37 | 1.3 | -- | 15-20 |
| I-131 | 9.1 | 2.9 | 3.1 | 33-86 | 10-40 |
| Sn-117m | 133-296 | 12-27 | 11 | ∼ 1000 | 5-10 |
| Tc-99m | 0.23 | 0.034 | 6.8 | -- | -- |

| | | | | | |
|---|---|---|---|---|---|
| Sr as the chloride; Re as HEDP, Sm as EDTMP; P as orthophosphate; I as BDP3; Sn as (4+)DTPA; Tc as MDP | | | | | |

to the quality of the patient's life than total sedation. As a further consequence, the use of addictive drugs is significantly reduced.

It has also been found, surprisingly and unexpectedly, that the present invention provides specific compositions using Sn-117m complexes which are highly effective in treating cancer in human skeletal bone whilst eliciting no significant or minimal toxicity. These advantages have been realized by discovering a unique pharmaceutical composition of this invention, and the results found herein have not been observed with treatments and/or compositions outside the parameters of the present invention.

It is an advantage of the present invention that the localization of the administered dose can be monitored by gamma scintigraphy. This physical characteristic of Sn⁴⁺-117m allows an integrated clinical approach to pain palliation and cancer radiotherapeutics, because it enables the physician to administer a particular dose to the site of the neoplastic lesion, to monitor localization of the radiopharmaceutical, to maintain a clinically effective dose at the neoplastic site, and to avoid bone marrow and normal tissue toxicity by keeping the level of radioisotope beneath toxic levels in non-skeletal tissues.

The methods for making and labeling these compounds are more fully illustrated in the following Examples, wherein Example 1 describes the preparation of Sn-117m; Example 2 describes the preparation of the Sn-117m-chelate complex; Example 3 describes preparation of Sn-117m-containing pharmaceutical compositions; Example 4 describes assays of radiochemical and radiopharmaceutical purity of the Sn-117m chelate; Example 5 demonstrates palliation of skeletal bone pain in human cancer patients. These Examples illustrate certain aspects of the above-described method and advantageous results. These Examples are shown by way of illustration and not by way of limitation.

### EXAMPLE 1

### Preparation of Sn-117m

Tin-117m was produced using the inelastic neutron scattering reaction:

¹¹⁷Sn(n,n'γ) → ^{117m}Sn.

Enriched Sn-117m (84%) as the oxide was obtained from Oak Ridge National Laboratory, Oak Ridge, TN. The oxide was converted to the metal by reduction at 600°C in a hydrogen flow for 2-5 hours. The metal was sealed in a quartz ampule to prepare a target (4-100 mg) for irradiation. Irradiation was carried out in the High Flux Beam Reactor at Brookhaven National Laboratory or the High Flux Isotope Reactor at Oak Ridge National Laboratory for periods up to 4 weeks.

### EXAMPLE 2

### Preparation of Sn-117m Chelate Complex

An ampule containing Sn-117m, prepared as described in Example 1, was opened in a nitrogen glove box and the contents dissolved in 0.2mL of concentrated hydrochloric acid with heat. The dissolved tin (as Sn²⁺) was then added to a 20-fold molar excess of the acid salt of DTPA that was previously adjusted to pH 6 with 3N sodium hydroxide. The pH was readjusted to 6, after the addition of tin, by using 3N and 1N sodium hydroxide. The solution was sterile filtered into a tared, sterile multi-injection bottle, which was capped and removed from the glove box. The bottle was weighed again to obtain a volume. The solution was heated by immersing the bottle in a bath of boiling water to complete complexation. After cooling, a 2-fold equivalent excess of 30% hydrogen peroxide was added to oxidize Sn²⁺ to Sn⁴⁺, and the sample again was heated for 5 minutes in a bath of boiling water and then cooled and transferred to the glove box.

### EXAMPLE 3

### Preparation of Sn-117m Pharmaceutical Composition

To a sample of Sn-117m DPTA, preparesd as described in Example 2 was added 80% of an equimolar amount (relative to DTPA) of calcium chloride dihydrate. The pH of the solution dropped from pH 6 to pH 4 and was not readjusted. The solution was filtered through a sterile 0.2 µm filter into a sterile multi-injection bottle, and the volume adjusted to 10 mL with sterile isotonic saline solution.

### EXAMPLE 4

### Assay of Pharmaceutical Acceptability

The sample as prepared in Example 3 was assayed for radioactivity and radiopurity with germanium gamma spectroscopy. Pyrogenicity was checked by using a limulus amebocyte lysate pyrogen kit (Whittaker Bioproducts, Walkersville, MD). Aliquots of the final product were spotted on chromatographic paper strips and were developed with a solution of 1 mg/mL DTPA, pH 5-6. Sn-117m (Sn⁴⁺) DTPA moves with the solvent front, whereas any hydrolyzed and/or uncomplexed tin remains at the origin. Tissue distribution in normal mice was determined for each run and compared with previously published data as an additional measure of quality control. Hydrolyzed tin, if present, results in a high percentage of uptake into the liver and spleen in mice.

### EXAMPLE 5

### Palliation of Skeletal Bone Pain in Human Cancer Patients

Fourteen patients were enrolled in a controlled clinical study, including 9 males and 5 females. The patients were administered an Sn-117m chelate complex-containing composition (described above in Example 3) sufficient to provide between 33 and 156 µCi/kg body weight. The protocol was approved by an institutional review board and the United States Food and Drug Administration (FDA). All subjects gave informed consent.

Prior to initiation of therapy a Tc-99m MDP bone imaging study was obtained. Complete blood counts, including a differential and platelet count, electrolytes, and blood chemistries were obtained. No radiotherapy or chemotherapy had been administered to the patients in the prior month. Hormonal therapy was continued if the patient had been on hormonal therapy for more than three months without improvement in their clinical status.

Sn-117m (Sn⁴⁺) DTPA was administered through an inlying infusion line over a period of 5-10 minutes. The patient was observed for two hours at which time a urine specimen was obtained. A complete urine collection was obtained over 4 days and a blood specimen obtained on the fourth day. This enabled an estimate of bone uptake. Two imaging studies to monitor the Sn-117m distribution were obtained in the first week and repeat chemistries were obtained at one week. Complete blood counts were obtained weekly for at least two months and less frequently thereafter. A repeat Tc-99m MDP bone imaging study was performed at two months.

Patients maintained a record of pain levels and sites daily for two weeks and then twice a week for the next 6 weeks. They were provided forms to record these data as well as to maintain a record of medications.

The results of this study are shown in the Table provided below. The data provided in the table show dose-dependent relief or palliation of pain resulting from single administrations of compositions containing Sn-117m (Sn⁴⁺) DTPA according to the invention.

As is indicated in the Table, several patients either died or were required to leave the treatment regimen. No data on pain relief were obtained from these patients (Patients 1-3 and 5). Other patients, having been given a lower does of the Sn-117m chelate composition, showed variable response to the treatment, in one case indicating that no relief was had. In clinical terms, the responses of the patients at relatively low dosages is promising but not predictable enough to be practicable.

The predictability of response is greatly improved, however, by using a higher dose range. For example, it is clear that of the seven patients who received more than about 9 mCi/70 kg (∼ 129 µCi/kg, or an administered dose greater than 7.4 mCi) all experienced relief of pain: five experienced excellent relief and two experienced partial relief. Thus, a dosage of at least about 9 mCi to about 25 mCi per 70 kg of body weight provides not only effective, but predictable, palliation of pain in cancer patients. Accordingly, it is preferred that the treatment of bone pain using compositions containing Sn-117m chelates be performed using between about 9 mCi and about 25 mCi per 70 kg of body weight.

In addition, no patients in the study showed evidence of hemopoietic toxicity. An exemplary data set obtained from one patient, Patient 11, shows no decrease in white blood cell (WBC) count or platelet count over three months following the single administration of Sn-117m chelate (10.4 mCi). As a result, a dosage of the Sn-117m chelate within the range found to be effective for consistent pain reduction can also be expected to be non-toxic to the patient.

Thus, it is another particular advantage of this invention that patients can be treated for palliation of bone pain without causing concomitant bone marrow toxicity, which toxicity can result in often life-threatening lowering of white blood cell (leukocyte), platelet, and hemoglobin levels in the blood. Other bone palliation agents, such as Sr-89, are know to cause bone marrow suppression (myelosuppression) at doses effective in palliating bone pain, thereby greatly reducing their therapeutic efficacy.

Patients having compromised bone marrow due to previous chemotherapy or radiation therapy can be treated for palliation of bone pain with Sn-117m without the risk of further, possibly catastrophic, deterioration in bone marrow functioning. Also, patients having received Sn-117m for palliation of bone pain are competent to receive further chemotherapy or radiation therapy, as their bone marrow integrity is not compromised by Sn-117m treatment. These advantages are not shared with other known bone pain palliating agents.

**TABLE III**

| PALLIATION OF BONE PAIN IN HUMAN SUBJECTS | | | | | | |
|---|---|---|---|---|---|---|
| Patient | Primary Cancer | Patient Weight kg | Total Administered | | % Total in Bone | Result |
| | | | mCi | mCi/kg | | |
| 1 | breast | 80 | 4.65 | 0.058 | | NA |
| 2 | breast | 53 | 1.76 | 0.033 | | NA |
| 3 | lung | 56 | 3.95 | 0.071 | | NA |
| 4 | prostate | 61 | 4.70 | 0.077 | | - |
| 5 | lung | 60 | 4.19 | 0.070 | | NA |
| 6 | unknown | 108 | 8.41 | 0.078 | | + |
| 7 | prostate | 59 | 4.95 | 0.084 | | ++ |
| 8 | lung | 61 | 8.95 | 0.146 | | ++ |
| 9 | breast | 59 | 8.41 | 0.142 | 72.1 | ++ |
| 10 | breast | 59 | 8.00 | 0.135 | 59.2 | ++ |
| 11 | prostate | 67 | 10.46 | 0.156 | 58.0 | + |
| 12 | prostate | 93 | 14.46 | 0.156 | 34.01 | ++ |
| 13 | prostate | 118 | 15.49 | 0.131 | 71.3 | ++ |
| 14 | breast | 50 | 7.54 | 0.151 | 69.2 | + |

| | | | | | | |
|---|---|---|---|---|---|---|
| NA = Data not available due to patient death or need for supplemental therapeutic treatment Categories of Pain Relief: - = No relief; + = Partial Relief; + + = Good Relief | | | | | | |

## Claims

1. A pharmaceutical composition comprising a Sn-117m (Sn⁴⁺) chelate complex and a pharmaceutically-acceptable carrier for use in
(a) palliating pain in skeletal bone associated with cancer in a human; or
(b) treating primary or metastatic cancer in skeletal bone in a human;
the composition having a specific activity of Sn-117m of about 0.1 mCi/mg to about 80 Ci/mg, the pharmaceutical composition further comprising a dosage of from about 10 mCi to about 50 mCi Sn-117m per 70 kilograms body weight.

2. The pharmaceutical composition of claim 1 wherein the Sn-117m (Sn⁴⁺) chelate complex is comprised of a chelating agent selected from the group consisting of methylenediphosphonate, pyrophosphate, ethylenedihydroxydisodium phosphonate, diethylenetriaminepentaacetic acid, and mixtures thereof.

3. The pharmaceutical composition of claim 2 wherein the Sn-117m complex comprises Sn-117m (Sn⁴⁺)-DTPA.

4. The pharmaceutical composition of any preceding claim that is administered at a dosage of about 10 to about 30 mCi Sn-117m per 70 kilograms body weight, preferably at a dosage of about 12 to about 25 mCi Sn-117m per 70 kilograms body weight.

5. The pharmaceutical composition of any preceding claim having a specific activity of from about 2 to about 20 mCi/mg Sn-117m, preferably having a specific activity of from about 2 to about 17 mCi/mg Sn-117m, more preferably having a specific activity of from about 2 to about 12 mCi/mg Sn-117m

6. The pharmaceutical composition of claim 3 comprising about a ten-fold to about a 45-fold molar excess of DTPA relative to total tin, preferably comprising about a 15-fold to about a 30-fold molar excess of DTPA relative to total tin, more preferably comprising about a twenty-fold molar excess of DTPA relative to total tin.

7. A pharmaceutical composition of any preceding claim further comprising a toxicity control agent, wherein said agent comprises a divalent calcium ion and reduces toxicity associated with administration of the Sn-117m (Sn⁴⁺) chelate complex and wherein if the Sn-117m complex comprises Sn-117m(4⁺) DTPA preferably the toxicity control agent comprises about 60% to about 90% of the molar amount of DTPA in the composition, more preferably about 70% to about 90% of the molar amount of DTPA in the composition, optionally about 80% of the molar amount of DTPA in the composition.

8. The pharmaceutical composition of claim 7 wherein the toxicity control agent is calcium chloride dihydrate (CaCl₂*2H₂O)..

9. The pharmaceutical composition of claim 7 having a specific activity of about 2 to about 20 mCi/mg in the case of (a) and of about 12 to about 20 mCi/mg in the case of (b) comprised of about a twenty-fold molar excess of DTPA to total tin and further comprising a toxicity control agent that is a divalent calcium ion in an amount that is about 80% of the molar amount of DTPA in the composition, wherein the pharmaceutical composition is administered at a dosage of from about 12 to about 25 mCi per 70 kilograms body weight in the case of (a) and at a dosage of from about 12 to about 20 mCi per 70 kilograms body weight in the case of (b).

10. Use of a composition of any preceding claim for preparing a medicament for
(a) palliating pain in skeletal bone associated with cancer in a human; or
(b) treating primary or metastatic cancer in skeletal bone in a human.

11. A method for manufacturing a medicament intended for
(a) palliating pain in skeletal bone associated with cancer in a human; or
(b) treating primary or metastatic cancer in skeletal bone in a human;
**characterized in that** a composition as defined in any one of claims 1 to 9 is used.

12. A pharmaceutical composition comprising a stannic chelate of tin-117m (Sn⁴⁺) for use in
(a) palliating pain in skeletal bone associated with cancer in a human; or
(b) treating primary or metastatic cancer in skeletal bone in a human;
the composition having a specific activity of Sn-117m of about 0.1 mCi/mg to about 80 Ci/mg, the pharmaceutical composition further comprising a dosage of from about 10 mCi to about 50 mCi Sn-117m per 70 kilograms body weight.

13. The use of a stannic chelate of tin-117m having a specific activity of Sn-117m of about 0.1 mCi/mg to about 80 Ci/mg for the manufacture of a medicament for
(a) palliating pain in skeletal bone associated with cancer in a human; or
(b) treating primary or metastatic cancer in skeletal bone in a human,
wherein the medicament is administered at a dosage of about 10 mCi to about 50 mCi Sn-117m per 70 kilograms of body weight.

## Patentansprüche

1. Pharmazeutische Zusammensetzung, umfassend einen Sn-117m (Sn⁴⁺)-Chelatkomplex und einen pharmazeutisch zulässigen Träger, zur Verwendung für die
(a) Linderung von Schmerzen in den Knochen des Skeletts im Zusammenhang mit einer Krebserkrankung bei einem Menschen; oder
(b) Behandlung einer primären oder metastasierten Krebserkrankung der Knochen des Skeletts bei einem Menschen;
wobei die Zusammensetzung eine spezifische Aktivität des Sn-117m von etwa 0,1 mCi/mg bis etwa 80 Ci/mg aufweist, und wobei die pharmazeutische Zusammensetzung ferner eine Dosierung von etwa 10 mCi bis etwa 50 mCi Sn-117m pro 70 kg Körpergewicht umfasst.

2. Pharmazeutische Zusammensetzung nach Anspruch 1, wobei der Sn-117m (Sn⁴⁺)-Chelatkomplex einen Chelatbildner umfasst, der aus der Gruppe, bestehend aus Methylendiphosphonat, Pyrophosphat, Ethylendihydroxydinatriumphosphonat, Diethylentriaminpentaessigsäure und Mischungen daraus ausgewählt ist.

3. Pharmazeutische Zusammensetzung nach Anspruch 2, wobei der Sn-117m-Komplex Sn-117m (Sn⁴⁺)-DTPA umfasst.

4. Pharmazeutische Zusammensetzung nach einem der vorhergehenden Ansprüche, welche in einer Dosierung von etwa 10 bis etwa 30 mCi Sn-117m pro 70 kg Körpergewicht, vorzugsweise in einer Dosierung von etwa 12 bis etwa 25 mCi Sn-117m pro 70 kg Körpergewicht verabreicht wird.

5. Pharmazeutische Zusammensetzung nach einem der vorhergehenden Ansprüche mit einer spezifischen Aktivität von etwa 2 bis etwa 20 mCi/mg Sn-117m, vorzugsweise mit einer spezifischen Aktivität von etwa 2 bis etwa 17 mCi/mg Sn-117m und mehr bevorzugt mit einer spezifischen Aktivität von etwa 2 bis etwa 12 mCi/mg Sn-117m.

6. Pharmazeutische Zusammensetzung nach Anspruch 3, welche einen etwa 10- bis etwa 45-fachen molaren Überschuss DTPA bezogen auf die Gesamtmenge an Zinn umfasst, vorzugsweise einen etwa 15- bis etwa 30-fachen molaren Überschuss DTPA bezogen auf die Gesamtmenge an Zinn umfasst und mehr bevorzugt einen etwa zwanzigfachen molaren Überschuss DTPA bezogen auf die Gesamtmenge an Zinn umfasst.

7. Pharmazeutische Zusammensetzung nach einem der vorhergehenden Ansprüche, welche ferner ein Toxizitätskontrollmittel umfasst, wobei das Mittel ein bivalentes Calciumion umfasst und die Toxizität im Zusammenhang mit der Verabreichung des Sn-117m (Sn⁴⁺)-Chelatkomplexes reduziert, und wobei, wenn der Sn-117m-Komplex Sn-117m(4⁺)-DTPA umfasst, das Toxizitätskontrollmittel vorzugsweise etwa 60 % bis etwa 90 % der molaren Menge an DTPA in der Zusammensetzung umfasst, mehr bevorzugt etwa 70 % bis etwa 90 % der molaren Menge an DTPA in der Zusammensetzung und optional etwa 80 % der molaren Menge an DTPA in der Zusammensetzung.

8. Pharmazeutische Zusammensetzung nach Anspruch 7, wobei das Toxizitätskontrollmittel Calciumchlorid Dihydrat (CaCl₂*2H₂O) ist.

9. Pharmazeutische Zusammensetzung nach Anspruch 7 mit einer spezifischen Aktivität von etwa 2 bis etwa 20 mCi/mg im Fall (a) und von etwa 12 bis etwa 20 mCi/mg im Fall (b), welche einen etwa zwanzigfachen molaren Überschuss DTPA bezogen auf die Gesamtmenge an Zinn umfasst und ferner ein Toxizitätskontrollmittel umfasst, bei dem es sich um ein bivalentes Calciumion in einer Menge, die etwa 80 % der molaren Menge an DTPA in der Zusammensetzung beträgt, handelt, wobei die pharmazeutische Zusammensetzung in einer Dosierung von etwa 12 bis etwa 25 mCi pro 70 kg Körpergewicht im Fall (a) und in einer Dosierung von etwa 12 bis etwa 20 mCi pro 70 kg Körpergewicht im Fall (b) verabreicht wird.

10. Verwendung einer Zusammensetzung nach einem der vorhergehenden Ansprüche für die Zubereitung eines Medikaments zur
(a) Linderung von Schmerzen in den Knochen des Skeletts im Zusammenhang mit einer Krebserkrankung bei einem Menschen; oder
(b) Behandlung einer primären oder metastasierten Krebserkrankung der Knochen des Skeletts bei einem Menschen.

11. Verfahren zur Herstellung eines Medikaments, vorgesehen zur
(a) Linderung von Schmerzen in den Knochen des Skeletts im Zusammenhang mit einer Krebserkrankung bei einem Menschen; oder
(b) Behandlung einer primären oder metastasierten Krebserkrankung der Knochen des Skeletts bei einem Menschen;
**dadurch gekennzeichnet, dass** eine Zusammensetzung, wie in einem der Ansprüche 1 bis 9 definiert, verwendet wird.

12. Pharmazeutische Zusammensetzung, welche ein Zinnchelat mit Zinn-117m (Sn⁴⁺) umfasst, für die Verwendung zur
(a) Linderung von Schmerzen in den Knochen des Skeletts im Zusammenhang mit einer Krebserkrankung bei einem Menschen; oder
(b) Behandlung einer primären oder metastasierten Krebserkrankung der Knochen des Skeletts bei einem Menschen,
wobei die Zusammensetzung eine spezifische Aktivität des Sn-117m von etwa 0,1 mCi/mg bis etwa 80 Ci/mg aufweist, und wobei die pharmazeutische Zusammensetzung ferner eine Dosierung von etwa 10 mCi bis etwa 50 mCi Sn-117m pro 70 kg Körpergewicht umfasst.

13. Verwendung eines Zinnchelats mit Zinn-117m mit einer spezifischen Aktivität des Sn-117m von etwa 0,1 mCi/mg bis etwa 80 Ci/mg für die Herstellung eines Medikaments zur
(a) Linderung von Schmerzen in den Knochen des Skeletts im Zusammenhang mit einer Krebserkrankung bei einem Menschen; oder
(b) Behandlung einer primären oder metastasierten Krebserkrankung der Knochen des Skeletts bei einem Menschen,
wobei das Medikament in einer Dosierung von etwa 10 mCi bis etwa 50 mCi Sn-117m pro 70 kg Körpergewicht verabreicht wird.

## Revendications

1. Composition pharmaceutique, comprenant un complexe de chélate Sn-117m (Sn⁴⁺) et un porteur pharmaceutiquement acceptable pour utilisation dans
(a) l'apaisement de douleurs dans l'os squelettique en rapport avec le cancer chez un être humain ; ou
(b) le traitement de cancer primaire ou métastatique dans l'os squelettique chez un être humain ;
la composition présentant une activité spécifique de Sn-117m d'environ 0,1 mCi/mg à environ 80 Ci/mg, la composition pharmaceutique comprenant en outre un dosage d'environ 10 mCi à environ 50 mCi Sn-117m pour 70 kilogrammes de poids du corps.

2. Composition pharmaceutique selon la revendication 1, dans laquelle le complexe de chélate Sn-117m (Sn⁴⁺) comprend un agent chélatant sélectionné dans le groupe formé par le méthylènediphosphonate, le pyrophosphate, le phosphonate d'éthylènedihydroxydisodium, l'acide diéthylènetriamine pentacétique, et des mélanges de ces derniers.

3. Composition pharmaceutique selon la revendication 2, dans laquelle le complexe Sn-117m comprend du Sn-117m (Sn⁴⁺)-DTPA.

4. Composition pharmaceutique selon l'une quelconque des revendications précédentes, administrée à un dosage d'environ 10 à environ 30 mCi Sn-117m pour 70 kilogrammes de poids du corps, de préférence à un dosage d'environ 12 à environ 25 mCi Sn-117m pour 70 kilogrammes de poids du corps.

5. Composition pharmaceutique selon l'une quelconque des revendications précédentes, présentant une activité spécifique d'environ 2 à environ 20 mCi/mg Sn-117m, présentant de préférence une activité spécifique d'environ 2 à environ 17 mCi/mg Sn-117m, présentant encore plus préférablement une activité spécifique d'environ 2 à environ 12 mCi/mg Sn-117m.

6. Composition pharmaceutique selon la revendication 3, comprenant environ un excès molaire de dix fois à 45 fois de DTPA par rapport à la quantité totale d'étain, comprenant de préférence un excès molaire d'environ 15 fois à environ 30 fois de DTPA par rapport à la quantité totale d'étain, comprenant encore plus préférablement un excès molaire d'environ vingt fois de DTPA par rapport à la quantité totale d'étain.

7. Composition selon l'une quelconque des revendications précédentes, comprenant en outre un agent de commande de toxicité, dans lequel ledit agent comprend un ion de calcium bivalent et réduit la toxicité associée à l'administration du complexe de chélate Sn-117m (Sn⁴⁺), et dans laquelle, si le complexe Sn-117m comprend du Sn-117m (4⁺)-DTPA, l'agent de commande de toxicité comprend de préférence environ 60% à environ 90% de la concentration moléculaire de DTPA dans la composition, encore plus préférablement environ 70% à environ 90% de la concentration moléculaire de DTPA dans la composition, en option environ 80% de la concentration moléculaire de DTPA dans la composition.

8. Composition pharmaceutique selon la revendication 7, dans laquelle l'agent de commande de toxicité est le dihydrate de chlorure de calcium (CaCl₂*2H₂O).

9. Composition pharmaceutique selon la revendication 7, présentant une activité spécifique d'environ 2 à environ 20 mCi/mg dans le cas (a) et d'environ 12 à environ 20 mCi/mg dans le cas (b), comprenant environ un excès molaire de vingt fois de DTPA par rapport à la quantité totale d'étain et comprenant en outre un agent de commande de toxicité qui est un ion de calcium bivalent en une concentration égale à environ 80% de la concentration moléculaire de DTPA dans la composition, dans laquelle la composition pharmaceutique est administrée à un dosage d'environ 12 à environ 25 mCi pour 70 kilogrammes de poids du corps dans le cas (a) et à un dosage d'environ 12 à environ 20 mCi pour 70 kilogrammes de poids du corps dans le cas (b).

10. Utilisation d'une composition selon l'une quelconque des revendications précédentes pour la préparation d'un médicament pour
(a) l'apaisement de douleurs dans l'os squelettique en rapport avec le cancer chez un être humain ; ou
(b) le traitement de cancer primaire ou métastatique dans l'os squelettique chez un être humain.

11. Méthode pour la fabrication d'un médicament destiné à/au
(a) l'apaisement de douleurs dans l'os squelettique en rapport avec le cancer chez un être humain ; ou
(b) traitement de cancer primaire ou métastatique dans l'os squelettique chez un être humain ;
**caractérisée en ce qu'**on utilise une composition conforme à la définition de l'une quelconque des revendications 1 à 9.

12. Composition pharmaceutique comprenant un chélate stannique d'étain-117m (Sn⁴⁺) pour utilisation dans
(a) l'apaisement de douleurs dans l'os squelettique en rapport avec le cancer chez un être humain ; ou
(b) le traitement de cancer primaire ou métastatique dans l'os squelettique chez un être humain ;
la composition présentant une activité spécifique de Sn-117m d'environ 0,1 mCi/mg à environ 80 Ci/mg, la composition pharmaceutique comprenant en outre un dosage d'environ 10 mCi à environ 50 mCi Sn-117m pour 70 kilogrammes de poids du corps.

13. Utilisation de chélate stannique d'étain-117m présentant une activité spécifique de Sn-117m d'environ 0,1 mCi/mg à environ 80 Ci/mg pour la fabrication d'un médicament pour
(a) l'apaisement de douleurs dans l'os squelettique en rapport avec le cancer chez un être humain ; ou
(b) le traitement de cancer primaire ou métastatique dans l'os squelettique chez un être humain,
dans laquelle le médicament est administré à un dosage d'environ 10 mCi à environ 50 mCi Sn-117m pour 70 kilogrammes de poids du corps.
